# EUROPEAN PATENT SPECIFICATION

(11) **EP 0 912 704 B1**
(45) Date of publication and mention of the grant of the patent: **04.06.2003**
(21) Application number: 97915471.3
(22) Date of filing: 29.03.1997
(51) Int. Cl.: C11D 17/00

(54) **SYSTEM FOR DELIVERY OF FUNCTION INGREDIENTS**
VERABREICHUNGSSYSTEM VON FUNKTIONELLEN WIRKSTOFFEN
SYSTEME D'APPORT D'INGREDIENTS FONCTIONNELS

(30) Priority: 24.05.1996 GB 9610966
(43) Date of publication of application: 06.05.1999
(73) Proprietor: UNILEVER PLC, London EC4P 4BQ (GB); UNILEVER N.V., 3013 AL Rotterdam (NL)
(72) Inventor: GOEL, Satish Kumar, Mumbia 400 099, Maharashtra (IN); SANKHOLKAR, Devadatta Shivaji, Mumbia 400 099, Maharashtra (IN)
(74) Representative: Rots, Maria Johanna Francisca
(86) International application number: EP9701624
(87) International publication number: WO97045525

(56) References cited:
- EP-A- 0 093 601
- EP-A- 0 552 024
- US-A- 3 580 853
- US-A- 5 250 652
- PATENT ABSTRACTS OF JAPAN vol. 010, no. 172 (C-354), 18 June 1986 & JP 61 022005 A (KOBAYASHI KOOSEE:KK), 30 January 1986,

## Description

The present invention relates to a novel system for enhanced delivery of functional ingredients for use in detergent soap bar formulations. In particular, the invention is directed to enhancing the deposition of benefit agents such as organic sunscreens, emollients, humectants, antimicrobial agents and insect repellants onto skin, hair or other substrates from detergent soap bar based washing compositions.

It is presently known in the art to use natural polysaccharide type polymers modified by adding cationic groups (for example, Jaguar, a registered trade name of Rhone Poulenc for cationic derivative of guar gum) as well as synthetic cationic polymers in washing formulations for providing better skin and hair feel. There are several patents describing their use as conditioners in liquid or gel type products. For instance, US 4,061,602 (American Cyanamide Co.) describes them as ingredients which add substance to hair and skin without building up after successive applications. EP 311,343 (P&G) suggests their use in solid soap bars for giving benefits related to mildness, scum-control and lather. The method of incorporation of Jaguar into the final product in these cases is by way of adding the Jaguar powder directly with the other ingredients of the product and mixing it thoroughly. US 4,704,224 (P&G) describes a better method of incorporating Jaguar in soap bars which is by way of preparing a small adjunct where Jaguar is dispersed in coconut fatty acid and neutralized separately before adding it to the rest of the soap. US 803742 (P&G) describes yet another method involving hydration of Jaguar with water prior to mixing it uniformly into the soap bars to give improved feel and mildness effect.

US 3,723,325 (P&G), South Africa 6805, 954 (P&G), EP 93,601 (Unilever), WO 9522311 (Unilever), WO 93EP2072 (Unilever) and GB 8212687 (Unilever) all describe the use of Jaguar and cationic polymers to enhance deposition of particulate solid benefit agents onto skin from washing compositions (mainly soap bars). WO 9403152 (Unilever) describes a similar application however, for deposition of silicone oil instead of solid particles. However, irrespective of the intended benefit agent used for delivery, all the above discussed known art methods are directed to the incorporation of Jaguar/cationic polymer by simply mixing it as powder in the formulation.

It is thus the basic objective of the present invention to provide for a novel system of enhanced delivery of functional ingredients for use in detergent soap bar formulations.

A further object of the present invention is to provide for suitable method(s) of incorporation of the system for enhanced delivery of the functional ingredient in detergent soap bar formulations.

Yet a further object of the present invention is to provide synergistic detergent soap bar formulations which would provide for improved functional ingredient delivery onto substrates on which they are applied thereby obtaining maximum effective value of the benefit agents used in such bar formulations.

Another object of the present invention is directed to provide for a detergent soap bar formulations with enhanced benefit agent depositing properties in a form which would be convenient for the user.

Thus, according to one aspect, the present invention provides a detergent soap bar composition according to claim 1 appended hereto. The composition includes a system for enhanced delivery of a benefit agent. The system comprises a synergistic oil-in-water type emulsion with an internal oil phase of said benefit agent and an external water phase thickened with cationic polymer.

The functional ingredient/benefit agent and the cationic polymer, when incorporated as said oil-in-water type emulsion in soaps/detergent based washing compositions, provide for enhanced deposition of the benefit agent such as organic sunscreen, Parsol MCX (a registered trade name of Givaudan Roure and chemically 2-ethyl-hexyl-methoxy cinnamate), Parsol 1789 (chemically known as butyl methoxy benzoylmethane) onto skin, hair and other substrate from soaps or detergent based washing compositions.

Suitable benefit agents for use in such emulsion systems are organic sun screen, emollients, humectants, antimicrobial agents and insect repellants.

The external water phase in the emulsion system is thickened by the addition of cationic polymers which includes JAGUAR (a tradename for cationic guar gum which is 2-hydroxy-3-(trimethylammonio)propyl guar gum). The cationic polymer is used in the range of 0.25 to 5% and preferably 1-2% based on the external water phase.

The oil-in-water emulsion system has an internal oil phase of more than 50% to give higher concentration of deposition of said benefit agent.

The oil-in-water type of emulsion system of the invention described above can be obtained as follows:
a. providing a solution of the cationic polymer in water the polymer being present in an amount of from 0.25-5%, based on the external water phase;
b. Mixing a suitable emulsifier preferably having HLB~4-10 with the selected benefit agent/functional ingredient.
c. The mixture in (b) is next subjected to high shear mixing preferably in a Silverson mixer while the solution in (a) is added slowly to it, to thereby obtain the benefit agent as oil-in-water type emulsion system of the invention wherein the mixture from step (b) is present in an amount greater than 50%.

According to another aspect of the present invention is provided a detergent /soap/washing composition formulations having improved functional ingredient depositing characteristic by way of incorporation of the benefit agent in the form of said oil-in-water type emulsion system in such bar formulations.

In accordance with such aspect of the invention the benefit agent in the form of the oil-in-water emulsion system is incorporated in the basic soap/detergent composition formulation which is selected from:
anionic surfactants such as sodium or potassium salts of fatty acids of varying chain lengths (soaps), sodium linear alkyl benzene sulphonate, alpha olefin sulphonate, sodium lauryl ether sulphate and primary alkyl sulphates in the range of about 10 to 80%;
non-ionic surfactants such as alkyl alcohol ethoxylates in the range of about 2 to 20%; and other surfactants such as cationic betaines in the range of about 2 to 10%; particulate minerals such as clays, talc and calcite in the range of about 0 to 40%;
soda ash in the range of about 0 to 10%;
builders such as sodium phosphates or zeolites in the range of about 0 to 20%;
binders such as alkaline sodium silicate in the range of about 0 to 5%; or
other optional minor ingradients such as perfumes, bleaches, optical brighteners and enzymes; or mixtures thereof.

The benefit agents used are organic sunscreens, emollients, humectants, antimicrobial agents and insect repellants, preferably in the range of about 0.1 to 5%. The benefit agent in the range of 0-1-5% is provided in the form of the oil-in-water emulsion in said bar formulation to achieve improved functional ingredient deposition characteristics in such bar formulations.

According to yet further aspect of the present invention the same is directed to providing for suitable methods of incorporation of the benefit agent/functional ingredients in the form of the oil-in-water type emulsion system of the invention in product bar fomulation such as soaps/detergent bars/washing compositions to obtain effective deposition of the functional ingredients/benefit agent onto substrates on which such products (bar formulations) are applied.

In particular, the invention thus further proposes incorporation of the oil-in-water type emulsion system containing the benefit agent into the final product in the form of any one or more of the following:
i) discrete domains of the emulsion (equivalently of the benefit agent since the emulsion has high concentration of the benefit agent);
ii) adding of the benefit agent in the form of the emulsion as stripes on the extruded soap bars preferably by injection facility on the plodder; and
iii) filing in the benefit agent in the form of the emulsion in a separate zone of a divided tube dispenser such that the benefit agent in the form of said emulsion flows out as stripes adhering to the main ribbon of a gel product as the tube is squeezed.

The nature of the present invention, its objectives and advantages will be further apparent from the ensuing description made with relation to non-limiting exemplary modes of obtaining the benefit agent in the form of oil-in-water type emulsion system of the invention and its incorporation in exemplary bar formulations as discussed hereunder.

### EXAMPLE 1

### (A) Preparation of emulsion

In this example, 1% solution of Jaguar in water was first prepared using an overhead stirrer. An emulsifier (Tween 60) having HLB~10 was mixed with a combination of benefit agents comprising Parsol MCX with Parsol 1789 (80:20 proportions).

This mixture of sunscreen with the emulsifier was next subjected to high shear mixing using a Silverson mixer while the Jaguar solution was added slowly to it. Mixing was continued for about 5 minutes after complete addition of Jaguar solution to homogenise the emulsion. The composition of the emulsion was Parsol mix: 55.6%, Tween 60: 7.5%, and 1% Jaguar solution: 36.9%.

### (B) Incorporation of oil-in-water emulsion of (A) above, into the product - by way of incorporation of the benefit agent in the form of speckled bars.

The emulsion system thus obtained in step (A) above was incorporated at 1.8% into a soap base comprising 30% coconut oil fatty acid salt, the balance being the salts of distilled fatty acids derived from a mixture of oils (eg. rice bran/palm) of moisture content 13%. The emulsion was incorporated into the product by very light blending of the emulsion with soap base in a ribbon mixer for just less than a minute followed by compaction of mix under pressure.

The level of incorporation of emulsion was 3.6% and the average level of Parsol mix in the product was 2%.

### EXAMPLE II

### (A) Preparation of the emulsion

In this example, 1% solution of Jaguar in water was first prepared using an overhead stirrer. An emulsifier (Tween 60) having HLB~10 was mixed with a combination of benefit agents comprising Parsol MCX with Parsol 1789 (80:20 proportions).

This mixture of sunscreen with the emulsifier was next subjected to high shear mixing using a Silverson mixer while . the Jaguar solution was added slowly to it. Mixing was continued for about 5 minutes after complete addition of Jaguar solution homogenise the emulsion. The composition of the emulsion was the same as in (A) of Example I.

### (B) Incorporation of oil-in-water emulsion of (A) above into the product - by way of incorporation of the benefit agent in the form of striped bars.

The oil-in-water type emulsion system of (A) above was next incorporated into the product bar formulation similar to that of Example I in the form of stripes by die injection technique so that the average level of Parsol mix in the product was 2.5%.

### EXAMPLE III

### Control bar formulation

In this example, a control bar formulation was made from the soap base containing 30% coconut oil fatty acid salt, the balance being the salts of distilled fatty acids derived from a mixture of oils (e.g., rice bran/palm) of moisture content of 13% without incorporation of any Parsol mix.

The deposition of Parsol onto the skin in use of the bar formulations of the invention (Examples I and II) and the control bar formulation (Example III) was estimated.

The estimation of Parsol deposition was carried out on 9 panelists following the under-mentioned procedure:

Twenty 'to and fro' rubs of the bar were given on prewashed inner forearm, the product was rubbed with the other hand to generate lather for 10 seconds, followed by rinsing, dab drying, and extraction with 400 micro liters of alcohol from 9 cm². The results of a comparative study of deposition from bar formulations according to Examples I to II vis-a-vis the control bar formulation III are provided hereunder in Tables I and II.

Comparative study of Parsol deposition from bar formulation according to Example I vis-a-vis Example III (control) is provided hereunder Table I.

**Table I**

| Deposition of Parsol (Micrograms on 9cm²)* | | |
|---|---|---|
| Example I | Example III | Difference |
| 3.07 | 2.30 | 0.77⁺ |

| | | |
|---|---|---|
| * Data are average of 9 values. | | |
| ⁺ Significant at 95% confidence. | | |

The above Table I shows that with 95% statistical confidence Parsol MCX is deposited from the bar formulation (Example I) of the invention onto the skin to the extent of 0.77 ± 0.27 micrograms on 9 cm² (ie., at least 0.50 microgram/9cm²). Comparative study of Parsol deposition from bar formulation according to Example II vis-a-vis Example III (control) is provided hereunder in Table II.

**Table II**

| Deposition of Parsol (Micrograms on 9cm²)* | | |
|---|---|---|
| Example II | Example III | Difference |
| 2.94 | 1.72 | 1.22⁺ |

| | | |
|---|---|---|
| * Data are average of 9 values. | | |
| ⁺ Significant at 95% confidence. | | |

The above Table II shows that with 95% confidence Parsol MCX is deposited from the bar formulations (Example II) of the invention onto the skin to the extent of 1.22 ± 0.38 micrograms on 9 cm² (i.e., at least 0.84 microgram on 9 cm²).

It is thus evident from the above results in Tables I and II that Parsol in water emulsions of satisfactory stability could be obtained wherein the aqueous phase is stabilized by cationic polymer such as Jaguar and that these emulsions could be used to generate macro domains of the benefit agent such as Parsol in soap/detergent bar formulations. Importantly, a comparative study of Tables I and II further reveal that at 2% or more Parsol mix (80:20) a consistent and significant enhanced level of deposition on skin is achieved using the bar formulations (Example I and II) of the invention as compared to control formulation (Example III).

### Product Assessment:

The product assessment of the bars having bar formulations according to Example I obtained by the process of the invention were next assessed vis-a-vis the control bar formulations according to Example III. The results are reproduced hereunder in Table III.

**Table III**

| Product attribute | Example III Control | Example I (2% Parsol) | C.D. |
|---|---|---|---|
| Lather, soft | 240 | 245 | 12.1 |
| Lather, hard | 193 | 191 | 14 |
| % Wear | 35.1 | 35.9 | 2.2 |
| Mush | 8.3 | 9.2 | 3.1 |

The above Table III clearly indicates that the product attributes of bar formulations according to Example I of the invention are comparable to the control bar formulation Example III.

It is thus clearly evident from the above disclosure that in the use of benefit agent in the form of oil-in-water emulsion system according to the invention in soap/detergent bar products in the manner proposed above, the deposition of benefit agent onto the skin/other substrate is significantly enhanced. Further such enhanced deposition of the benefit agent can be achieved following the process of incorporation of the same in detergent/soap bar formulations according to the invention even at very low levels of use of cationic polymer in combination with the benefit agent by way of the novel oil-in-water emulsion system of the invention.

## Claims

1. A detergent soap bar composition incorporating an oil-in-water emulsion system for enhanced delivery of benefit agent,comprising an internal oil phase comprising an organic sun screen, an emollient, a humectant, an antimicrobial agent or an insect repellant, wherein the oil phase is present in an amount greater than 50% by weight of the emulsion, and wherein an external water phase thickened with a cationic polymer is present in an amount of from 0.25 to 5%, based on the external water phase.

2. A bar composition as claimed in claim 1, wherein the cationic polymer is present at from 1 to 2%, based upon the external water phase.

3. A bar composition as claimed in any one of the preceding claims wherein the emulsion is incorporated in the form of discrete domains or stripes.

4. A bar composition as claimed in any one of claims 1 to 3, further comprising any of the following:
anionic surfactants such as sodium or potassium salts of fatty acids of varying chain lengths (soaps), sodium linear alkyl benzene sulphonate, alpha olefin sulphonate, sodium lauryl ether sulphate and primary alkyl sulphates in the range of about 10 to 80%;
non-ionic surfactants such as alkyl alcohol ethoxylates in the range of about 2 to 20%; and other surfactants such as cationic betaines in the range of about 2 to 10%;
particulate minerals such as clays, talc and calcite in the range of about 0 to 40%;
soda ash in the range of about 0 to 10%;
builders such as sodium phosphates or zeolites in the range of about 0 to 20%;
binders such as alkaline sodium silicate in the range of about 0 to 5%; or
other optional minor ingredients such as perfumes, bleaches, optical brighteners and enzymes; or mixtures thereof.

5. A method of producing a bar composition as claimed in any preceding claim comprising the steps of:
a) providing a solution of cationic polymer in water, the polymer being present in an amount of from 0.25 to 5%, based on the external water phase;
b) mixing an emulsifier having HLB 4-10 with said benefit agent; and
c) subjecting the mixture resulting from step (b) to high shear mixing,
while adding the solution of step (a) wherein the mixture from step (b) is present in an amount greater than 50%.

6. Use of a bar composition in any of claims 1 to 4 in the effective deposition of benefit agent onto substrates on which said bars are applied.

## Patentansprüche

1. Waschmittelseifenriegelzusammensetzung, die zur verbesserten Abgabe von Vorteil-verleihendem Mittel ein Öl-in-Wasser-Emulsionssystem enthält, umfassend eine innere Ölphase, die ein organisches Sonnenschutzmittel, ein Erweichungsmittel, ein Feuchthaltemittel, ein antimikrobielles Mittel oder ein Insekten-abweisendes Mittel umfasst, wobei die Ölphase in einer Menge größer als 50 Gewichtsprozent der Emulsion vorliegt und wobei eine äußere Wasserphase, die mit einem kationischen Polymer verdickt ist, in einer Menge von 0,25 bis 5%, bezogen auf die äußere Wasserphase, vorliegt.

2. Riegelzusammensetzung nach Anspruch 1, wobei das kationische Polymer mit 1 bis 2%, bezogen auf die äußere Wasserphase, vorliegt.

3. Riegelzusammensetzung nach einem der vorangehenden Ansprüche, wobei die Emulsion in Form von diskreten Domänen oder Streifen eingearbeitet worden ist.

4. Riegelzusammensetzung nach einem der Ansprüche 1 bis 3, die weiterhin eines der Nachstehenden umfasst:
anionische Tenside, wie Natrium- oder Kaliumsalze von Fettsäuren mit variierenden Kettenlängen (Seifen), lineares Natrium-Alkylbenzolsulfonat, α-Olefinsulfonat, Natriumlaurylethersulfat und primäre Alkylsulface im Bereich von etwa 10 bis 80%;
nichtionische Tenside, wie Alkylalkoholethoxylate, im Bereich von etwa 2 bis 20%, und andere Tenside, wie kationische Betaine, im Bereich von etwa 2 bis 10%;
teilchenförmige Mineralien, wie Tone, Talkum und Calcit, im Bereich von etwa 0 bis 40%;
wasserfreie Soda im Bereich von etwa 0 bis 10%;
Builder, wie Natriumphosphate oder Zeolithe, im Bereich von etwa 0 bis 20%;
Bindemittel, wie alkalisches Natriumsilikat, im Bereich von etwa 0 bis 5%; oder
andere, wahlweise geringe Bestandteile, wie Parfüms, Bleichmittel, optische Aufheller und Enzyme, oder Gemische davon.

5. Verfahren zur Herstellung einer Riegelzusammensetzung nach einem vorangehenden Anspruch, umfassend die Schritte von:
a) Bereitstellen einer Lösung von kationischem Polymer in Wasser, wobei das Polymer in einer Menge von 0,25 bis 5%, bezogen auf die äußere Wasserphase, vorliegt;
b) Vermischen eines Emulgators mit einem HLB-Wert von 4-10 mit dem Vorteil-verleihenden Mittel; und
c) Unterziehen des aus Schritt (b) erhaltenen Gemisches Mischen bei hoher Scherwirkung,
unter Zugeben der Lösung von Schritt (a), wobei das Gemisch von Schritt (b) in einer Menge größer als 50% vorliegt.

6. Verwendung einer Riegel Zusammensetzung nach einem der Ansprüche 1 bis 4 bei der wirksamen Abscheidung von Vorteil-verleihendem Mittel auf Substrate, auf die die Riegel angewendet werden.

## Revendications

1. Composition de pain de savon détergent incorporant un système d'émulsion huile dans l'eau pour un apport amélioré d'agent bénéfique, comprenant une phase interne d'huile comprenant un écran solaire organique, un émollient, un humectant, un agent antimicrobien ou un agent répulsif contre les insectes, dans laquelle la phase d'huile est présente dans une quantité supérieure à 50 % en poids de l'émulsion, et dans laquelle une phase d'eau externe épaissie avec un polymère cationique qui est présent dans une quantité allant de 0,25 à 5 %, sur la base de la phase d'eau externe.

2. Composition de pain selon la revendication 1, dans laquelle le polymère cationique est présent à un niveau de 1 à 2 % sur la base de la phase d'eau externe.

3. Composition de pain selon l'une quelconque des revendications précédentes dans laquelle l'émulsion est incorporée sous la forme de bandes ou de domaines discrets.

4. Composition de pain selon l'une quelconque des revendications 1 à 3, comprenant en outre n'importe lequel des ingrédients suivants :
des tensioactifs anioniques tels que des sels de sodium ou de potassium d'acides gras ayant des longueurs de chaînes variables (savons), de l'alkyle benzène sulfonate linéaire de sodium, de l'alpha oléfine sulfonate, du lauryle éther sulfate de sodium et des alkyles sulfates primaires dans une quantité allant d'environ 10 à 80 % ;
des tensioactifs non ioniques tels que des éthoxylats d'alcool alkylique dans une quantité allant d'environ 2 à 20 % ;
et d'autres tensioactifs tels que des bétaines cationiques dans une quantité allant d'environ 2 à 10 % ;
des minéraux particulaires tels que des argiles, du talc et de la calcite dans une quantité allant d'environ 0 à 40 % ;
du carbonate de soude dans une quantité allant d'environ 0 à 10 % ;
des édificateurs tels que des phosphates de sodium ou des zéolites dans une quantité allant d'environ 0 à 20 % ;
des liants tels que du silicate de sodium alcalin dans une quantité allant d'environ 0 à 5 % ; ou
d'autres ingrédients optionnels mineurs tels que des parfums, des blanchissants, des aviveurs optiques et des enzymes ; ou des mélanges de ceux-ci.

5. Procédé de production d'une composition de pain selon l'une quelconque des revendications précédentes, comprenant les étapes consistant à :
(a) fournir une solution de polymère cationique dans de l'eau, le polymère étant présent dans une quantité allant de 0,25 à 5 %, sur la base de la phase d'eau externe;
(b) mélanger avec ledit agent bénéfique un émulsifiant ayant une valeur HLB de 4 à 10 ;
(c) soumettre le mélange résultant de l'étape (b) à un mélange à cisaillement élevé,
tout en ajoutant la solution obtenue dans le cadre de l'étape (a) dans laquelle le mélange obtenu dans le cadre de l'étape (b) est présent dans une quantité supérieure à 50 %.

6. Utilisation d'une composition de pain selon l'une quelconque des revendications 1 à 4 pour déposer de manière efficace un agent bénéfique sur des substrats sur lesquels sont appliqués lesdits pains.
